(19) **Europäisches Patentamt / European Patent Office / Office européen des brevets**

(11) **EP 2 399 523 A1**

(12) # EUROPEAN PATENT APPLICATION
published in accordance with Art. 153(4) EPC

(43) Date of publication:
**28.12.2011 Bulletin 2011/52**

(51) Int Cl.:
**A61B 10/00** (2006.01)   **A61B 8/08** (2006.01)
**G01N 21/17** (2006.01)

(21) Application number: **10743616.4**

(22) Date of filing: **22.01.2010**

(86) International application number:
**PCT/JP2010/050801**

(87) International publication number:
**WO 2010/095487 (26.08.2010 Gazette 2010/34)**

(84) Designated Contracting States:
**AT BE BG CH CY CZ DE DK EE ES FI FR GB GR HR HU IE IS IT LI LT LU LV MC MK MT NL NO PL PT RO SE SI SK SM TR**

(30) Priority: **23.02.2009 JP 2009039709**

(71) Applicant: **Olympus Medical Systems Corp.
Tokyo 151-0072 (JP)**

(72) Inventor: **IGARASHI Makoto
Tokyo 151-0072 (JP)**

(74) Representative: **Gunzelmann, Rainer
Wuesthoff & Wuesthoff
Patent- und Rechtsanwälte
Schweigerstraße 2
D-81541 München (DE)**

(54) **ORGANISM OBSERVATION DEVICE AND ORGANISM TOMOGRAM CREATING METHOD**

(57)    A biomedical imaging apparatus according to the present invention includes: an ultrasound generating section configured to output ultrasound to a predetermined region in an object under examination; an illuminating light generating section configured to emit illuminating light to the predetermined region upon which the ultrasound is incident; a phase component detecting section configured to time-resolve return light of the illuminating light emitted to the predetermined region, from the first time point to the Nth time point, and thereby detect the first to the Nth phase components of the return light corresponding to the first time point to the Nth time point; and a computing section configured to perform a process for subtracting a sum of the first to the (N-1)th phase components from the Nth phase component based on the phase components detected by the phase component detecting section.

**FIG.1**

EP 2 399 523 A1

**Description**

Technical Field

The present invention relates to a biomedical imaging apparatus and a biomedical tomographic image generation method and, more particularly, to a biomedical imaging apparatus and a biomedical tomographic image generation method which acquire in-vivo information using sound waves and light in conjunction.

Background Art

In recent years, various techniques have been proposed to implement optical tomographic imaging of living bodies, the techniques including, for example, optical CT, optical coherence tomography (hereinafter abbreviated to OCT), and photoacoustic tomography.

Optical CT, which uses near-infrared light in the wavelength region of 700 to 1,200 nm relatively unaffected by light scattering in living bodies, can obtain tomographic images in a living body up to a depth of a few cm under a mucosa.

Also, OCT which uses interference can obtain biomedical tomographic images to a depth of about 2 mm at high resolutions (a few $\mu$m to ten-odd $\mu$m) in a short time. OCT is a technique which has already been put to practical use for diagnosis of retinal diseases in the field of ophthalmology, and is a subject of very high medical interest.

Although optical CT provides information about deep parts, it has a spatial resolution of as low as a few mm. On the other hand, with OCT, it is difficult to observe a depth of 2 mm or more under a living mucosa, and to obtain high image quality in the case of tumor tissue such as cancer.

To deal with this, a technique is disclosed in Japanese Patent Application Laid-Open Publication No. 2007-216001. The technique visualizes normal tissue and tumor tissue such as cancer by detecting results of interaction between light and ultrasound in a living mucosa as amounts of change in phase components of light.

Also, a technique related to ultrasound-modulated optical tomography is disclosed by C. Kim, K.H. Song, L.V. Wang in "Sentinel lymph node detection ex vivo using ultrasound-modulated optical tomography," J. Biomed. Opt. 13 (2), 2008 (hereinafter referred to as "non-patent document"). The technique is capable of obtaining tomographic images in deep parts of a living body at a higher spatial resolution than optical CT by detecting light modulated by ultrasound emitted to living tissue.

By the way, when emitting ultrasound and illuminating light to a desired position in a biological medium and obtaining biomedical information based on an object beam generated at the desired position, observed amounts of phase components of the object beam change depending on amounts of change of phase components received from a biological medium existing on paths of the illuminating light and the object beam. For this reason, when generating a tomographic image of a desired region using only observed amounts of phase components of an object beam as in the technique recited in, e.g. Japanese Patent Application Publication No. 2007-216001, in some cases a (blurry) image is generated with which it is difficult to distinguish between a normal tissue and a tumor tissue such as cancer. Further, in the technique recited in Japanese Patent Application Publication No. 2007-216001, no proposal is made to eliminate such problem.

Furthermore, the technique recited in the above-mentioned non-patent document is for performing imaging specialized for a light-absorbing object of various biological media. As such, the technique recited in the above-mentioned non-patent document has a problem of being incapable of generating an image that allows clear distinction between a normal tissue and tumor tissue such as cancer.

The present invention was made in view of the above described circumstances, and has an objective to provide a biomedical imaging apparatus and biomedical tomographic image generation method that enable a normal tissue and a tumor tissue such as cancer to be visualized in high contrast.

Disclosure of Invention

Means for Solving the Problem

The present invention provides a biomedical imaging apparatus comprising: an ultrasound generating section configured to output ultrasound to a predetermined region in an object under examination; an illuminating light generating section configured to emit illuminating light to the predetermined region upon which the ultrasound is incident; a phase component detecting section configured to time-resolve return light of the illuminating light emitted to the predetermined region, from the first time point to the Nth time point, and thereby detect the first to the Nth phase components of the return light corresponding to the first time point to the Nth time point; and a computing section configured to perform a process for subtracting a sum of the first to the (N-1)th phase components from the Nth phase component based on the phase components detected by the phase component detecting section.

The present invention provides a biomedical tomographic image generation method comprising the steps of: outputting ultrasound to a predetermined region in an object under examination; emitting illuminating light to the predetermined region upon which the ultrasound is incident; time-resolving return light of the illuminating light emitted to the predetermined region, from the first time point to the Nth time point, and thereby detecting the first to the Nth phase components of the return light corresponding to the first time point to the Nth time point; performing a process for subtracting a sum of the first to the (N-1)th phase components from the Nth phase component based on the phase components detected by the phase component detecting section; and generating a tomographic image of the predetermined region using process results of the process as a pixel component.

Brief Description of the Drawings

Fig. 1 is a diagram showing an exemplary principal configuration of an optical imaging apparatus according to an embodiment of the present invention;
Fig. 2 is a flowchart showing an example of processes performed by the optical imaging apparatus in Fig. 1;
Fig. 3 is a schematic diagram showing a case in which an object beam is generated at the (j+1)th depth location from a surface of living tissue;
Fig. 4 is a diagram showing an exemplary principal configuration, different from the one in Fig. 1, of an optical imaging apparatus according to the embodiment of the present invention;
Fig. 5 is a diagram showing a detailed configuration around an optical coupler in Fig. 4;
Fig. 6 is a diagram showing an exemplary configuration of an edge of an optical fiber included in the optical imaging apparatus in Fig. 4; and
Fig. 7 is a flowchart showing an example of processes performed by the optical imaging apparatus in Fig. 4.

Best Mode for Carrying Out the Invention

An embodiment of the present invention will be described with reference to the drawings.

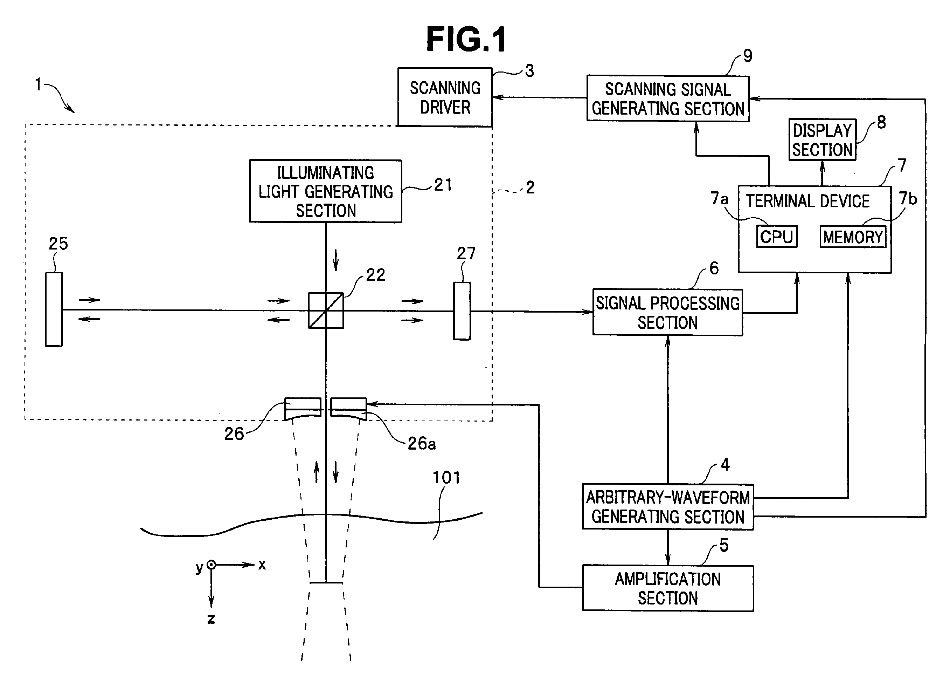

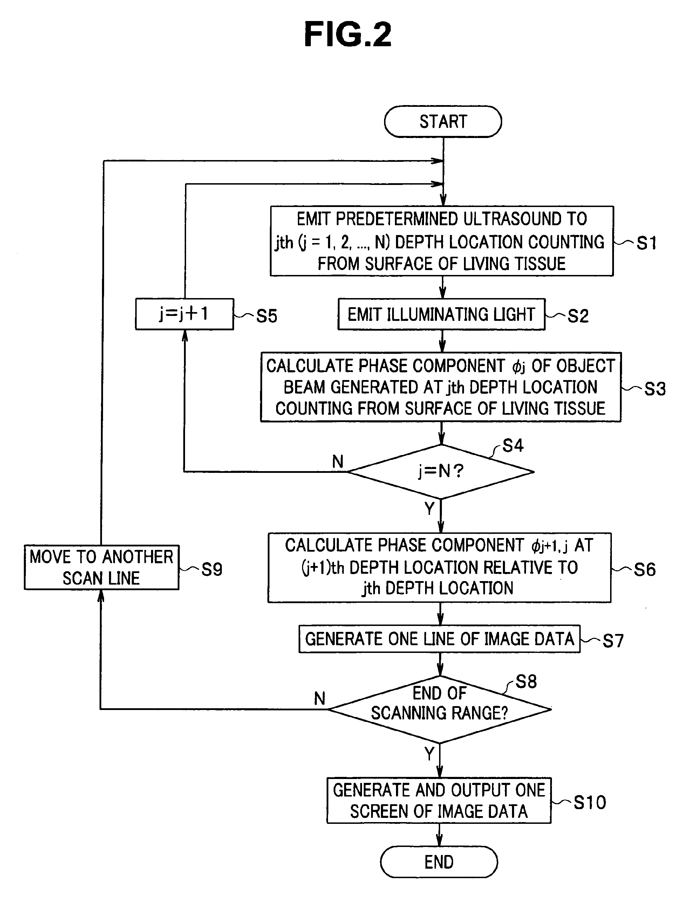

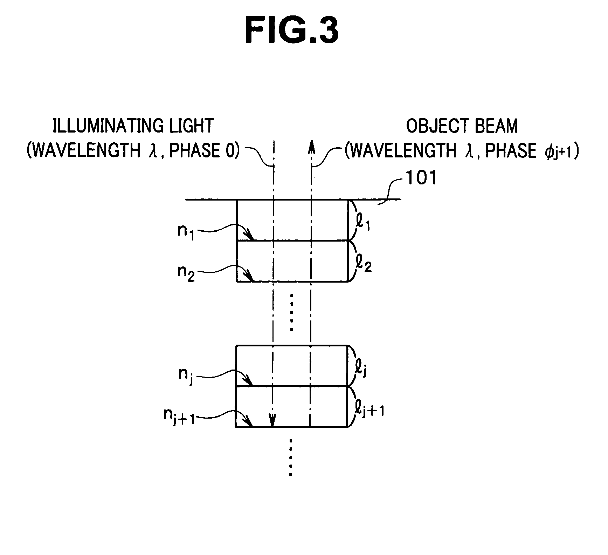

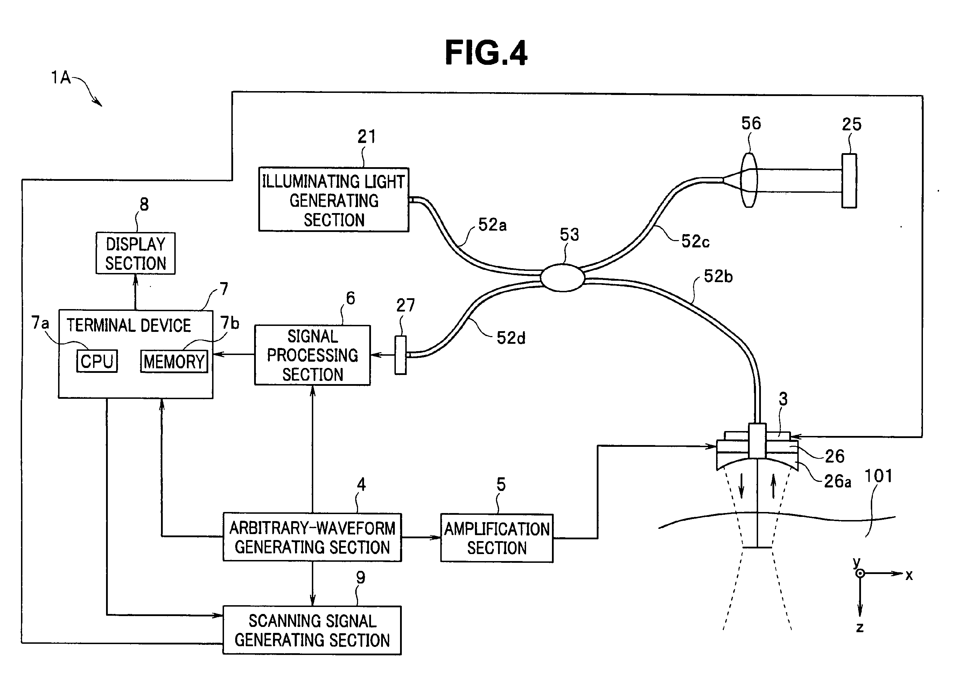

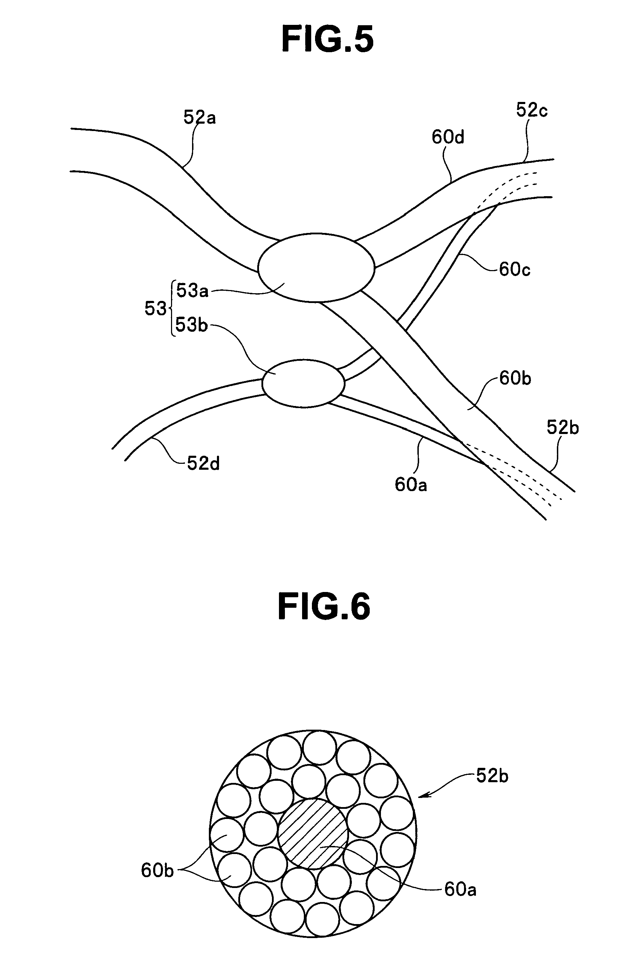

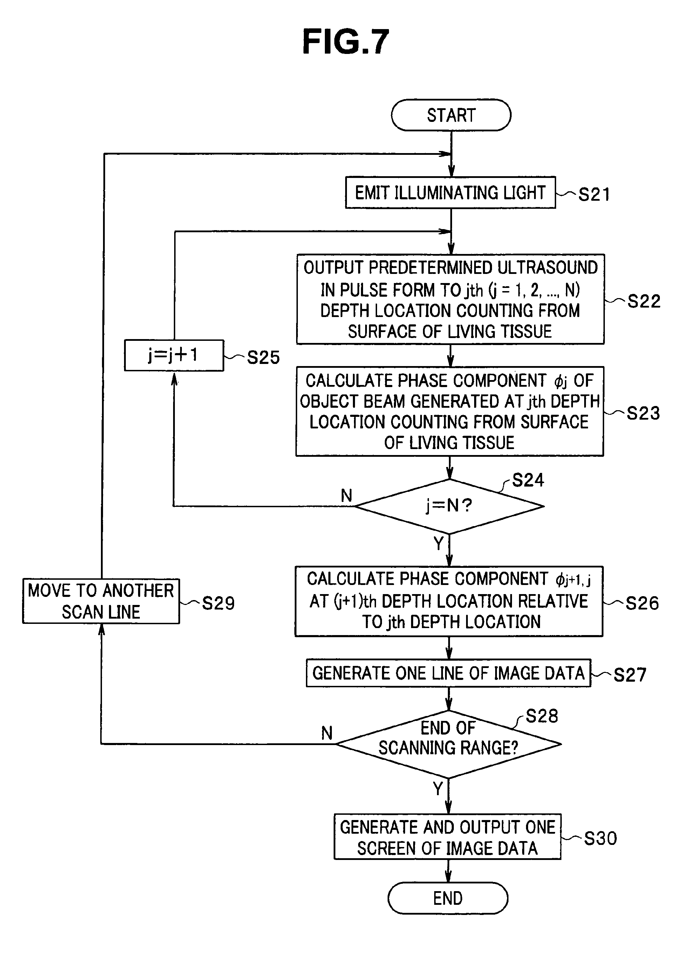

Figs. 1 to 7 concern the embodiment of the present invention. Fig. 1 is a diagram showing an exemplary principal configuration of an optical imaging apparatus according to the embodiment of the present invention. Fig. 2 is a flowchart showing an example of processes performed by the optical imaging apparatus in Fig. 1. Fig. 3 is a schematic diagram showing a case in which an object beam is generated at the (j+1)th depth location from a surface of living tissue. Fig. 4 is a diagram showing an exemplary principal configuration, different from the one in Fig. 1, of an optical imaging apparatus according to the embodiment of the present invention. Fig. 5 is a diagram showing a detailed configuration around an optical coupler in Fig. 4. Fig. 6 is a diagram showing an exemplary configuration of an edge of an optical fiber included in the optical imaging apparatus in Fig. 4. Fig. 7 is a flowchart showing an example of processes performed by the optical imaging apparatus in Fig. 4.

As shown in Fig. 1, an optical imaging apparatus 1 as a biomedical imaging apparatus includes a unit 2, a scanning driver 3, an arbitrary-waveform generating section 4, an amplification section 5, a signal processing section 6, a terminal device 7, a display section 8, and a scanning signal generating section 9, where the unit 2 emits ultrasound and illuminating light to a living tissue 101 which is an object under examination and can receive an object beam which is the illuminating light reflected and scattered by the living tissue 101, the scanning driver 3 causes the ultrasound and the illuminating light to be emitted by changing position of the unit 2 (scan position) according to a scanning signal outputted from the scanning signal generating section 9, and the display section 8 is made up of a monitor and the like.

The unit 2 includes an illuminating light generating section 21, a half mirror 22, a reference mirror 25, an ultrasound transducer 26, an acoustic lens 26a, and a light detection section 27, where an opening portion is formed through centers of the ultrasound transducer 26 and acoustic lens 26a.

The illuminating light generating section 21 is a laser source, or a combination of an SLD (Super Luminescent Diode) or a while light source and interference filters, capable of generating coherent light as illuminating light reachable to the object under examination in the living tissue 101. The illuminating light emitted from the illuminating light generating section 21 is not limited to continuous light, and may be, for example, pulsed light.

The half mirror 22 reflects part of the illuminating light coming from the illuminating light generating section 21 and emits the illuminating light to the reference mirror 25 while transmitting other part of the illuminating light through the half mirror 22 to the ultrasound transducer 26.

The illuminating light emitted from the half mirror 22 to the reference mirror 25 is reflected by the reference mirror 25 and then becomes incident on the half mirror 22 as a reference beam.

The illuminating light transmitted through the half mirror 22 to the ultrasound transducer 26 is emitted to the living tissue 101 through the opening portion provided in the centers of the ultrasound transducer 26 and acoustic lens 26a.

According to the present embodiment, it is assumed that space between the unit 2 (on the side of acoustic lens 26a) and the living tissue 101 has been filled with an ultrasound transmission medium such as water when a process for obtaining biomedical information about the living tissue 101 is performed by various parts of the optical imaging apparatus 1.

On the other hand, based on an ultrasound drive signal from the arbitrary-waveform generating section 4, the ultrasound transducer 26 emits predetermined ultrasound which is a continuous wave to the living tissue 101 along an optical axis of the illuminating light passing through the opening portion. The predetermined ultrasound emitted from the ultrasound transducer 26 propagates in the living tissue 101 as a periodic compressional wave while being converged by the acoustic lens 26a, and then converges in a predetermined region in a depth direction (z-axis direction in Fig. 1) of the living tissue 101.

The acoustic lens 26a is configured such as to be able to change, as appropriate, the region in which the predetermined ultrasound converges in the depth direction (z-axis direction in Fig. 1) of the living tissue 101, for example, under the control of the scanning driver 3.

On the other hand, the illuminating light emitted from the unit 2 is reflected at a location corresponding to the region in which the predetermined ultrasound converges, out of locations in the depth direction (z-axis direction in Fig. 1) of the living tissue 101, passed through the opening portion in the centers of the ultrasound transducer 26 and acoustic lens 26a, and becomes incident on the half mirror 22 as an object beam (return light). That is, the illuminating light transmitted through the half mirror 22 is reflected in the living tissue 101 at a location where density of the living tissue 101 is increased by the predetermined ultrasound. Then, the illuminating light becomes incident on the half mirror 22 as an object beam.

Then, the half mirror 22 causes two fluxes of the reference beam incident from the reference mirror 25 and the object beam incident from the ultrasound transducer 26 to interfere with each other and emits resulting interfering light to the light detection section 27.

The light detection section 27 heterodyne-detects the interfering light emitted from the half mirror 22, converts the detected interfering light into an interference signal which is an electrical signal, and outputs the interference signal to the signal processing section 6.

Each time a scanning signal is inputted from the scanning signal generating section 9, the scanning driver 3 changes positions of the ultrasound transducer 26 and acoustic lens 26a in an x-axis direction or y-axis direction in Fig. 1.

The arbitrary-waveform generating section 4 outputs an ultrasound drive signal to the amplification section 5 to make the ultrasound transducer 26 and the acoustic lens 26a output predetermined ultrasound of a predetermined wavelength (or predetermined frequency). Also, the arbitrary-waveform generating section 4 outputs a timing signal to the scanning signal generating section 9, indicating output timing of the ultrasound drive signal to the amplification section 5. Furthermore, the arbitrary-waveform generating section 4 outputs a trigger signal to the terminal device 7 and the scanning signal generating section 9 when an end of a scanning range is reached for the scanning driver 3. Furthermore, the arbitrary-waveform generating section 4 outputs the timing signal to the signal processing section 6 with a delay of a predetermined time.

The amplification section 5 made up of a power amplifier or the like amplifies the ultrasound drive signal outputted from the arbitrary-waveform generating section 4 and outputs the amplified ultrasound drive signal to the ultrasound transducer 26.

The signal processing section 6 equipped with a spectrum analyzer, a digital oscilloscope, or the like (none is shown) detects the interference signal outputted from the light detection section 27. Then, the signal processing section 6 time-resolves detection results of the interference signal based on the timing signal from the arbitrary-waveform generating section 4, thereby acquires observed amounts of phase components, and then outputs the observed amounts of phase components to the terminal device 7.

The terminal device 7 made up of a computer and the like includes a CPU 7a which performs various computing operations and processes as well as a memory 7b.

The CPU 7a calculates relative amounts of the phase components at locations in the depth direction of the living tissue 101, excluding an outermost layer, based on the observed amounts of the phase components outputted from the signal processing section 6.

Also, based on the observed amounts of the phase components in the outermost layer of the living tissue 101 and calculation results of the relative amounts of the phase components, the CPU 7a generates image data line by line along the depth direction of the living tissue 101, with N pixels contained in each line, and accumulates the generated image data line by line in the memory 7b.

Then, upon detecting that the scanning has been completed based on the trigger signal outputted from the arbitrary-waveform generating section 4, the CPU 7a reads M lines of image data accumulated in the memory 7b during the period from input of the previous trigger signal to input of the current trigger signal and thereby generates one screen of image data including N pixels in a vertical direction and M pixels in a horizontal direction. Subsequently, the CPU 7a converts the one screen of image data into a video signal and outputs the video signal to the display section 8. Consequently, the display section 8 displays an internal image (tomographic image) of the living tissue 101, for example, in an x-z plane out of coordinate axes shown in Fig.1.

Each time a timing signal and a trigger signal are inputted from the arbitrary-waveform generating section 4, the scanning signal generating section 9 outputs a scanning signal to the scanning driver 3 to change the scan position.

Next, operation of the optical imaging apparatus 1 according to the present embodiment will be described.

After turning on various parts of the optical imaging apparatus 1, the user places the ultrasound transducer 26 (and acoustic lens 26a) such that ultrasound and illuminating light will be emitted in the z-axis direction in Fig. 1 (depth direction of the living tissue 101) at one scan position and fills the space between the ultrasound transducer 26 (and acoustic lens 26a) and the living tissue 101 with an ultrasound transmission medium such as water.

Subsequently, the user gives a command to start acquiring biomedical information from the living tissue 101, for example, by turning on a switch or the like in an operation section (not shown).

Based on the command from the operation section (not shown), the arbitrary-waveform generating section 4 outputs an ultrasound drive signal to the ultrasound transducer 26 via the amplification section 5 in order to output predetermined ultrasound.

Based on the inputted ultrasound drive signal, the ultrasound transducer 26 and the acoustic lens 26a emit the predetermined ultrasound to the jth (j = 1, 2, ..., N) depth location counting from a surface of the living tissue 101 along an emission direction of the illuminating light (Step S 1 in Fig. 2). Consequently, the predetermined ultrasound emitted from the ultrasound transducer 26 and the acoustic lens 26a propagates in the living tissue 101 as a periodic compressional wave and converges at the jth depth location counting from the surface of the living tissue 101. According to the present embodiment, it is assumed that the index value j of the depth location counting from the surface of the living tissue 101 is set at intervals of one pixel in an output image.

After the predetermined ultrasound is emitted from the ultrasound transducer 26 and the acoustic lens 26a, the illuminating light is emitted from the illuminating light generating section 21 to the half mirror 22 (Step S2 in Fig. 2).

The illuminating light emitted from the illuminating light generating section 21 is emitted in the z-axis direction in Fig. 1 (depth direction of the living tissue 101) through the opening portion provided in the centers of the ultrasound transducer 26 and acoustic lens 26a after passing through the half mirror 22, the reference mirror 25, and the like. In the following description, it is assumed that the illuminating light emitted through the opening portion has a phase of 0.

The illuminating light emitted to the living tissue 101 is reflected at the jth depth location counting from the surface of the living tissue 101. Then, after passing through the opening portion in the centers of the ultrasound transducer 26 and acoustic lens 26a, the illuminating light becomes incident on the half mirror 22 as an object beam.

The object beam incident from the ultrasound transducer 26 interferes on the half mirror 22 with the reference beam incident from the reference mirror 25, and resulting interfering light becomes incident on the light detection section 27.

The light detection section 27 heterodyne-detects the interfering light emitted from the half mirror 22, converts the detected interfering light into an interference signal which is an electrical signal, and outputs the interference signal to the signal processing section 6.

The signal processing section 6 which functions as a phase component detecting section acquires a phase component $\phi_j$ of the object beam generated at the jth depth location counting from the surface of the living tissue 101 (Step S3 in Fig. 2), time-resolves the object beam based on input timing of a timing signal from the arbitrary-waveform generating section 4, thereby associates the phase component $\phi_j$ with the index value j of the depth location, and temporarily accumulates a value of the phase component $\phi_j$.

Subsequently, various parts of the optical imaging apparatus 1 repeats Steps S 1 to S3 in Fig. 2 until the phase component $\phi_N$ of the object beam generated at the Nth depth location counting from the surface of the living tissue 101 is acquired (Steps S4 and S5 in Fig. 2).

That is, as Steps S 1 to S3 in Fig. 2 are repeated, ultrasound is incident on different depth locations from the first to the Nth depth locations counting from the surface of the living tissue 101 and illuminating light is emitted from the illuminating light generating section 21 in sequence at different time points from the first time point to the Nth time point. Consequently, the values of the phase components $\phi_1$, $\phi_2$, ..., $\phi_{N-1}$, $\phi_N$ are temporarily accumulated in the signal processing section 6 by being associated with the index values 1, 2, ..., N-1, N of the depth locations.

The illuminating light reflected from the first depth location counting from the surface of the living tissue 101, i.e., the outermost layer of the living tissue 101, becomes incident on the half mirror 22 as an object beam having the phase component $\phi_1$. Let $n_1$ denote a refractive index at the first depth location counting from the surface of the living tissue 101, let $l_1$ denote a distance (physical length) to the first depth location counting from the surface of the living tissue 101, and let $\lambda$ denote wavelength of the illuminating light, then the phase component $\phi_1$ is given by Equation (1) below.

$$\phi_1 = 2 \cdot 2\pi \frac{n_1 l_1}{\lambda} \quad \cdot \cdot \cdot \quad (1)$$

Similarly, for example, as shown in Fig. 3, let $n_{j+1}$ denote a refractive index at the (j+1)th depth location counting from the surface of the living tissue 101, let $l_{j+1}$ denote a distance (physical length) from the jth depth location to the j+1 depth location counting from the surface of the living tissue 101, and let $\lambda$ denote the wavelength of the illuminating light (and object beam), then the phase component $\phi_{j+1}$ of the object beam as return light from the j+1 depth location counting from the surface of the living tissue 101 is given by Equation (2) below.

$$\phi_{j+1} = 2 \cdot 2\pi \left( \frac{n_1 l_1}{\lambda} + \frac{n_2 l_2}{\lambda} + \cdots + \frac{n_j l_j}{\lambda} + \frac{n_{j+1} l_{j+1}}{\lambda} \right) \quad \cdot \cdot \cdot \quad (2)$$

Thus, the phase component $\phi_{j+i}$ acquired by the signal processing section 6 contains values corresponding to the phase components $\phi_1$, $\phi_2$, ..., $\phi_j$.

After acquiring the phase component $\phi_N$ of the object beam generated at the Nth depth location counting from the surface of the living tissue 101, the signal processing section 6 associates the phase component $\phi_N$ with the index value N by time-resolving the object beam. Subsequently, the signal processing section 6 outputs the values of the phase components $\phi_1$, $\phi_2$, ..., $\phi_{N-1}$, $\phi_N$ associated with the index values 1, 2, ..., N-1, N of the depth locations to the terminal device 7, as acquired results of the observed amounts of the phase components.

Based on the observed amounts of the phase components outputted from the signal processing section 6, the CPU 7a which functions as a computing section subtracts the phase component $\phi_j$ obtained at the jth depth location adjacent to the (j+1)th depth location from the phase component $\phi_{j+i}$ obtained at the (j+1)th depth location and thereby calculates a phase component $\phi_{j+1,j}$ at the (j+1)th depth location relative to the jth depth location using Equation (3) below (Step S6 in Fig. 2). In other words, the CPU 7a performs the process of calculating a sum total of amounts of change in phase components undergone by the illuminating light incident through the surface of the living tissue 101 until the illuminating light reaches the jth depth location and amounts of change in phase components undergone by the object beam after passing through the jth depth location until the object beam reaches the surface of the living tissue 101 and subtracting the phase component $\phi_j$ equivalent to the calculated sum total from the phase component $\phi_{j+1}$ obtained at the (j+1)th depth location corresponding to the location under examination. Consequently, values of $\phi_{2,1}$, $\phi_{3,2}$, ..., $\phi_{N,N-1}$ corresponding to relative amounts of the phase components are calculated.

$$\phi_{j+1,j} = \phi_{j+1} - \phi_j$$

$$= 2 \cdot 2\pi \left\{ \left( \frac{n_1 l_1}{\lambda} + \frac{n_2 l_2}{\lambda} + \cdots + \frac{n_j l_j}{\lambda} + \frac{n_{j+1} l_{j+1}}{\lambda} \right) - \left( \frac{n_1 l_1}{\lambda} + \frac{n_2 l_2}{\lambda} + \cdots + \frac{n_j l_j}{\lambda} \right) \right\}$$

$$= 2 \cdot 2\pi \frac{n_{j+1} l_{j+1}}{\lambda} \quad \cdot \cdot \cdot \quad (3)$$

Then, using, as a pixel component, the value of the phase component $\phi_1$ at the first depth location counting from the surface of the living tissue 101 and the values of the phase components $\phi_{2,1}$, $\phi_{3,2}$, ..., $\phi_{N,N-1}$ at the second to the Nth depth locations counting from the surface of the living tissue 101, the CPU 7a generates one line of image data made up of N pixels along the depth direction of the living tissue 101 (Step S7 in Fig. 2). In this way, the CPU 7a accumulates image data line by line in the memory 7b.

Incidentally, the pixel component used by the CPU 7a according to the present embodiment to generate one line of image data is not limited to the value of the phase component $\phi_1$ and the values of the phase components $\phi_{2,1}$, $\phi_{3,2}$, ..., $\phi_{N,N-1}$, and the CPU 7a may alternatively use values of refractive indexes $n_1$, $n_2$, ..., $n_{N-1}$, $n_N$ contained in the phase components.

Based on whether or not a trigger signal has been inputted from the arbitrary-waveform generating section 4, the CPU 7a determines whether or not the scan line used to acquire one line of image data in Step S7 in Fig. 2 is the end of the scanning range for the scanning driver 3 (Step S8 in Fig. 2).

If the scan line is not the end of the scanning range for the scanning driver 3 (scanning has not been completed), the CPU 7a moves to another scan line (different from the previous scan line in either the x-axis direction or y-axis direction in Fig. 1) by controlling the scanning signal generating section 9 (Step S9 in Fig. 2). Subsequently, the operation described above is repeated by various parts of the optical imaging apparatus 1 until the scan line reaches the end of the scanning range for the scanning driver 3.

Upon detecting completion of scanning based on input of a trigger signal, the CPU 7a reads M lines of image data accumulated in the memory 7b during the period from the previous trigger signal input to the current trigger signal input and thereby generates one screen of image data including N pixels in the vertical direction and M pixels in the horizontal direction. Subsequently, the CPU 7a converts the one screen of image data into a video signal and outputs the video signal to the display section 8 (Step S10 in Fig. 2). Consequently, the display section 8 displays an internal image (tomographic image) of the living tissue 101, for example, in the x-z plane out of the coordinate axes shown in Fig. 1.

As described above, in obtaining biomedical information based on an object beam generated at a desired location in a biological medium by emitting ultrasound and illuminating light to the desired location, the optical imaging apparatus 1 according to the present embodiment is configured to operate so as to be able to obtain the biomedical information at the desired location by removing amounts of change in phase components caused by the biological medium existing on paths of the illuminating light and the objective beam. Consequently, the optical imaging apparatus 1 according to the present embodiment visualizes normal tissue and tumor tissue such as cancer, which are biological media differing in refractive index from each other, with high contrast.

Incidentally, in acquiring the values of the phase components $\phi_1$, $\phi_2$, ..., $\phi_{N-1}$, $\phi_N$ on a scan line in the depth direction of the living tissue 101 by emitting ultrasound and illuminating light, it is not strictly necessary for the optical imaging apparatus 1 to be configured to start from the surface side and descend gradually deeper into the living tissue 101.

To provide advantages similar to those described above, the optical imaging apparatus 1 shown in Fig. 1 may also be configured, for example, as an optical imaging apparatus 1A shown in Fig. 4.

Specifically, the optical imaging apparatus 1A includes optical fibers 52a, 52b, 52c, and 52d, an optical coupler 53, and a collimating lens 56 in addition to the scanning driver 3, the arbitrary-waveform generating section 4, the amplification section 5, the signal processing section 6, the terminal device 7, the display section 8, the scanning signal generating section 9, the illuminating light generating section 21, the reference mirror 25, the ultrasound transducer 26, the acoustic lens 26a, and the light detection section 27.

The optical coupler 53 includes a first coupler section 53a and a second coupler section 53b as shown in Fig. 5.

The optical fiber 52a is connected at one end to the illuminating light generating section 21, and at the other end to the first coupler section 53a as shown in Figs. 5 and 6.

The optical fiber 52b includes a light-receiving fiber bundle 60a and a light-transmitting fiber bundle 60b as shown in Fig. 6. The fiber bundle 60a is connected at one end to the second coupler section 53b while the other end is passed through the opening portion formed in the centers of the ultrasound transducer 26 and acoustic lens 26a and connected to the opening. The fiber bundle 60b is connected at one end to the first coupler section 53a while the other end is passed through the opening portion formed in the centers of the ultrasound transducer 26 and acoustic lens 26a and connected to the opening. The ends of the fiber bundles 60a and 60b are placed in the opening portion formed in the centers of the ultrasound transducer 26 and acoustic lens 26a, for example, in a state shown in Fig. 6.

The optical fiber 52c includes a light-receiving fiber bundle 60c and a light-transmitting fiber bundle 60d as shown in Fig. 5. The fiber bundle 60c is connected at one end to the second coupler section 53b while the other end is placed such that light from the collimating lens 56 can be incident thereon. The fiber bundle 60d is connected at one end to the first coupler section 53a while the other end is placed so as to be able to emit light to the collimating lens 56.

The optical fiber 52d is connected at one end to the second coupler section 53b, and at the other end to the light detection section 27 as shown in Figs. 4 and 5.

With the configuration of the optical imaging apparatus 1A described above, the illuminating light from the illuminating light generating section 21 is emitted to the living tissue 101 via the optical fiber 52a, the first coupler section 53a, and the fiber bundle 60b and is emitted to the collimating lens 56 via the optical fiber 52a, the first coupler section 53a, and the fiber bundle 60d.

The illuminating light incident on the collimating lens 56 is emitted as light with a parallel light flux, reflected by the reference mirror 25, passed through the collimating lens 56 again, and then made incident on the fiber bundle 60c as a reference beam. The reference beam incident on the fiber bundle 60c is emitted to the second coupler section 53b.

On the other hand, the illuminating light emitted via the fiber bundle 60b is reflected at a location (the jth depth location counting from the surface of the living tissue 101) corresponding to the region in which predetermined ultrasound emitted from the ultrasound transducer 26 and acoustic lens 26a converges, out of locations in the depth direction (z-axis direction in Fig. 4) of the living tissue 101, and becomes incident on the fiber bundle 60a as an object beam.

The object beam incident from the fiber bundle 60a interferes in the second coupler section 53b with the reference beam incident from the fiber bundle 60c, producing interfering light. The interfering light becomes incident on the light detection section 27 through the optical fiber 52d.

Incidentally, the optical imaging apparatus 1A does not always need to be configured with the optical fiber 52b which incorporates the fiber bundle 60a and the fiber bundle 60b as shown in Fig. 6, and may be configured with a single optical fiber which serves both as a light-receiving fiber bundle and a light-transmitting fiber bundle.

Subsequently, processes similar to the series of processes illustrated in the flowchart in Fig. 2 are performed to generate image data line by line, with N pixels contained in each line, and thereby generate one screen of image data including N pixels in a vertical direction and M pixels in a horizontal direction.

Being configured to operate as described above, the optical imaging apparatus 1A visualizes normal tissue and tumor tissue such as cancer with high contrast as in the case of the optical imaging apparatus 1.

Incidentally, the advantages described above are provided not only by interference type systems such as exemplified in Figs. 1 and 4, but also by non-interference type systems.

Also, according to the present embodiment, the predetermined ultrasound emitted from the ultrasound transducer 26 and acoustic lens 26a is not limited to a continuous wave, and may be a pulsed wave.

In the example described below, it is assumed that in the optical imaging apparatus 1A shown in Fig. 4, the illuminating light emitted from the illuminating light generating section 21 is continuous light while the predetermined ultrasound emitted from the ultrasound transducer 26 and acoustic lens 26a is a pulsed wave.

After turning on various parts of the optical imaging apparatus 1A, the user places the ultrasound transducer 26 (and acoustic lens 26a) such that ultrasound and illuminating light will be emitted in the z-axis direction in Fig. 4 (depth direction of the living tissue 101) at one scan position and fills the space between the ultrasound transducer 26 (and acoustic lens 26a) and the living tissue 101 with an ultrasound transmission medium such as water.

Subsequently, the user gives a command to start acquiring biomedical information from the living tissue 101, for example, by turning on a switch or the like in an operation section (not shown).

Based on the command from the operation section (not shown), the illuminating light generating section 21 emits continuous light as illuminating light (Step S21 in Fig. 7).

The illuminating light emitted from the illuminating light generating section 21 is emitted in the z-axis direction in Fig. 4 (depth direction of the living tissue 101) through the optical fiber 52a, the first coupler section 53a, and the fiber bundle 60b.

On the other hand, after the illuminating light is emitted from the illuminating light generating section 21, the arbitrary-waveform generating section 4 outputs an ultrasound drive signal to the ultrasound transducer 26 via the amplification section 5 in order to output the predetermined ultrasound in pulse form.

Based on the inputted ultrasound drive signal, the ultrasound transducer 26 and the acoustic lens 26a output the predetermined ultrasound in pulse form to the jth (j = 1, 2, ..., N) depth location counting from the surface of the living tissue 101 along an emission direction of the illuminating light (Step S22 in Fig. 7).

Consequently, the predetermined ultrasound outputted in pulse form from the ultrasound transducer 26 and the acoustic lens 26a propagates in the living tissue 101 as a periodic compressional wave and converges at the jth depth location counting from the surface of the living tissue 101.

On the other hand, the illuminating light emitted to the living tissue 101 is reflected at the jth depth location counting from the surface of the living tissue 101 and becomes incident on the fiber bundle 60a as an object beam.

The object beam incident from the fiber bundle 60a interferes in the second coupler section 53b with the reference beam incident from the fiber bundle 60c, producing interfering light. The interfering light becomes incident on the light detection section 27 through the optical fiber 52d.

The light detection section 27 heterodyne-detects the interfering light emitted from the optical fiber 52d, converts the detected interfering light into an interference signal which is an electrical signal, and outputs the interference signal to the signal processing section 6.

The signal processing section 6 acquires the phase component $\phi_j$ of the object beam generated at the jth depth location counting from the surface of the living tissue 101 (Step S23 in Fig. 7). Then, the signal processing section 6 time-resolves the object beam based on the input timing of a timing signal from the arbitrary-waveform generating section 4, thereby associates the phase component $\phi_j$ with the index value j of the depth location, and temporarily accumulates values of the phase component $\phi_j$.

Subsequently, various parts of the optical imaging apparatus 1A repeats Steps S22 and S23 in Fig. 7 until the phase component $\phi_N$ of the object beam generated at the Nth depth location counting from the surface of the living tissue 101 is acquired (Steps S24 and S25 in Fig. 7).

That is, as Steps S22 and S23 in Fig. 7 are repeated, an object beam is generated each time the pulsed ultrasound is incident on a different depth location from the first to the Nth depth locations counting from the surface of the living tissue 101. Consequently, the values of the phase components $\phi_1, \phi_2, ..., \phi_{N-1}, \phi_N$ are temporarily accumulated in the signal processing section 6 by being associated with the index values 1, 2, ..., N-1, N of the depth locations.

Then, the signal processing section 6 acquires the phase component $\phi_N$ of the object beam generated at the Nth depth location counting from the surface of the living tissue 101, time-resolves the object beam, thereby associates the phase component $\phi_N$ with the index value N. Subsequently, the signal processing section 6 outputs the values of the phase components $\phi_1$, $\phi_2$, ..., $\phi_{N-1}$, $\phi_N$ associated with the index values 1,2, ..., N-1, N of the depth locations to the terminal device 7, as acquired results of the observed amounts of the phase components.

Based on the observed amounts of the phase components outputted from the signal processing section 6, the CPU 7a which functions as a computing section subtracts the phase component $\phi_j$ obtained at the jth depth location adjacent to the (j+1)th depth location from the phase component $\phi_{j+1}$ obtained at the (j+1)th depth location and thereby calculates a phase component $\phi_{j+1,j}$ at the (j+1)th depth location relative to the jth depth location using Equation (3) above (Step S26 in Fig. 7).

Then, using, as a pixel component, the value of the phase component $\phi_1$ at the first depth location counting from the surface of the living tissue 101 and the values of the phase components $\phi_{2,1}$, $\phi_{3,2}$, ..., $\phi_{N,N-1}$ at the second to the Nth depth locations counting from the surface of the living tissue 101, the CPU 7a generates one line of image data made up of N pixels along the depth direction of the living tissue 101 (Step S27 in Fig. 7). In this way, the CPU 7a accumulates image data line by line in the memory 7b.

Based on whether or not a trigger signal has been inputted from the arbitrary-waveform generating section 4, the CPU 7a determines whether or not the scan line used to acquire one line of image data in Step S27 in Fig. 7 is the end of the scanning range for the scanning driver 3 (Step S28 in Fig. 7).

If the scan line is not the end of the scanning range for the scanning driver 3 (scanning has not been completed), the CPU 7a moves to another scan line (different from the previous scan line in either the x-axis direction or y-axis direction in Fig. 4) by controlling the scanning signal generating section 9 (Step S29 in Fig. 7). Subsequently, the operation described above is repeated by various parts of the optical imaging apparatus 1A until the scan line reaches the end of the scanning range for the scanning driver 3.

Upon detecting completion of scanning based on input of a trigger signal, the CPU 7a reads M lines of image data accumulated in the memory 7b during the period from the previous trigger signal input to the current trigger signal input and thereby generates one screen of image data including N pixels in the vertical direction and M pixels in the horizontal direction. Subsequently, the CPU 7a converts the one screen of image data into a video signal and outputs the video signal to the display section 8 (Step S30 in Fig. 7). Consequently, the display section 8 displays an internal image (tomographic image) of the living tissue 101, for example, in an x-z plane out of coordinate axes shown in Fig. 4.

Thus, normal tissue and tumor tissue such as cancer can also be visualized with high contrast through the series of processes in Fig. 7.

The present invention is not limited to the embodiment described above, and various changes and alterations may be made without departing from the scope and spirit of the present invention.

The present application is filed claiming the priority of Japanese Patent Application No. 2009-039709 filed in Japan on February 23, 2009, and the above described disclosure is incorporated by reference in the present description, claims and drawings.

**Claims**

1. A biomedical imaging apparatus comprising:

   an ultrasound generating section configured to output ultrasound to a predetermined region in an object under examination;
   an illuminating light generating section configured to emit illuminating light to the predetermined region upon which the ultrasound is incident;
   a phase component detecting section configured to time-resolve return light of the illuminating light emitted to the predetermined region, from the first time point to the Nth time point, and thereby detect the first to the Nth phase components of the return light corresponding to the first time point to the Nth time point; and
   a computing section configured to perform a process for subtracting a sum of the first to the (N-1)th phase components from the Nth phase component based on the phase components detected by the phase component detecting section.

2. The biomedical imaging apparatus according to claim 1, wherein the computing section generates a tomographic image of the predetermined region using process results of the process as a pixel component.

3. The biomedical imaging apparatus according to claim 1 or 2, wherein the illuminating light is coherent light.

4. A biomedical tomographic image generation method comprising the steps of:

outputting ultrasound to a predetermined region in an object under examination;

emitting illuminating light to the predetermined region upon which the ultrasound is incident;

time-resolving return light of the illuminating light emitted to the predetermined region, from the first time point to the Nth time point, and thereby detecting the first to the Nth phase components of the return light corresponding to the first time point to the Nth time point;

performing a process for subtracting a sum of the first to the (N-1)th phase components from the Nth phase component based on the phase components detected by the phase component detecting section; and

generating a tomographic image of the predetermined region using process results of the process as a pixel component.

5. The biomedical tomographic image generation method according to claim 4, wherein the illuminating light is coherent light.

# FIG.1

SCANNING DRIVER — 3

SCANNING SIGNAL GENERATING SECTION — 9

DISPLAY SECTION — 8

ILLUMINATING LIGHT GENERATING SECTION — 21

1

2

25

27

22

26

26a

101

TERMINAL DEVICE — 7
7a CPU  7b MEMORY

SIGNAL PROCESSING SECTION — 6

ARBITRARY-WAVEFORM GENERATING SECTION — 4

AMPLIFICATION SECTION — 5

y ⊙ → x
z

EP 2 399 523 A1

# FIG.2

```
                              ┌──────────┐
                              │  START   │
                              └──────────┘
                                    │
                                    ▼
        ┌──────────────────────────────────────────────────┐
        │ EMIT PREDETERMINED ULTRASOUND TO                  │
        │ jth (j = 1, 2, ..., N) DEPTH LOCATION COUNTING    │──S1
        │ FROM SURFACE OF LIVING TISSUE                     │
        └──────────────────────────────────────────────────┘
                                    │
      ┌───────────┐        ┌──────────────────────┐
      │ j=j+1     │──S5    │ EMIT ILLUMINATING LIGHT │──S2
      └───────────┘        └──────────────────────┘
                                    │
        ┌──────────────────────────────────────────────────┐
        │ CALCULATE PHASE COMPONENT φj OF OBJECT           │
        │ BEAM GENERATED AT jth DEPTH LOCATION             │──S3
        │ COUNTING FROM SURFACE OF LIVING TISSUE           │
        └──────────────────────────────────────────────────┘
                                    │
                                    ▼       S4
                       N    ◇───────────────◇
                    ┌───────│     j=N?       │
                    │       ◇───────────────◇
                    │               │ Y
                    │               ▼
        ┌───────────────────┐  ┌──────────────────────────────┐
        │ MOVE TO ANOTHER   │  │ CALCULATE PHASE COMPONENT φj+1,j AT │
        │ SCAN LINE         │──S9  (j+1)th DEPTH LOCATION RELATIVE TO │──S6
        └───────────────────┘  │ jth DEPTH LOCATION            │
                    │          └──────────────────────────────┘
                    │                      │
                    │          ┌──────────────────────────────┐
                    │          │ GENERATE ONE LINE OF IMAGE DATA │──S7
                    │          └──────────────────────────────┘
                    │                      │
                    │              N       ▼       S8
                    │          ◇───────────────────◇
                    └──────────│   END OF           │
                               │ SCANNING RANGE?    │
                               ◇───────────────────◇
                                        │ Y
                                        ▼
                          ┌──────────────────────────┐
                          │ GENERATE AND OUTPUT ONE  │──S10
                          │ SCREEN OF IMAGE DATA     │
                          └──────────────────────────┘
                                        │
                                   ┌──────────┐
                                   │   END    │
                                   └──────────┘
```

# FIG.3

ILLUMINATING LIGHT
(WAVELENGTH $\lambda$, PHASE 0)

OBJECT BEAM
(WAVELENGTH $\lambda$, PHASE $\phi_{j+1}$)

101

$n_1$    $\ell_1$

$n_2$    $\ell_2$

$n_j$    $\ell_j$

$n_{j+1}$    $\ell_{j+1}$

# FIG.4

EP 2 399 523 A1

# FIG.5

# FIG.6

# FIG.7

```
                    ┌─────────────┐
                    │    START    │
                    └──────┬──────┘
                           │
                           ▼
                 ┌──────────────────────┐
                 │ EMIT ILLUMINATING    │──S21
                 │ LIGHT                │
                 └──────────┬───────────┘
                            │
                            ▼
              ┌──────────────────────────────┐
              │ OUTPUT PREDETERMINED         │
              │ ULTRASOUND IN PULSE FORM TO  │
              │ jth (j = 1, 2, ..., N)       │──S22
              │ DEPTH LOCATION COUNTING FROM │
              │ SURFACE OF LIVING TISSUE     │
              └──────────────┬───────────────┘
 ┌─────────┐                 │
 │ j=j+1   │──S25            ▼
 └─────────┘     ┌──────────────────────────────┐
                 │ CALCULATE PHASE COMPONENT φj │
                 │ OF OBJECT BEAM GENERATED AT  │
                 │ jth DEPTH LOCATION COUNTING  │──S23
                 │ FROM SURFACE OF LIVING TISSUE│
                 └──────────────┬───────────────┘
                                │
                                ▼      S24
                    N    ◇─────────────◇
                    ─────│   j = N ?   │
                         ◇─────────────◇
                                │ Y
                                ▼
              ┌──────────────────────────────┐
 ┌──────────────┐ │ CALCULATE PHASE COMPONENT   │
 │ MOVE TO     │  │ φj+1,j AT (j+1)th DEPTH     │──S26
 │ ANOTHER     │──│ LOCATION RELATIVE TO jth    │
 │ SCAN LINE   │S29│ DEPTH LOCATION             │
 └──────────────┘ └──────────────┬───────────────┘
                                │
                                ▼
              ┌──────────────────────────────┐
              │ GENERATE ONE LINE OF IMAGE   │──S27
              │ DATA                         │
              └──────────────┬───────────────┘
                                │
                                ▼       S28
                    N    ◇─────────────◇
                    ─────│  END OF     │
                         │ SCANNING    │
                         │ RANGE?      │
                         ◇─────────────◇
                                │ Y
                                ▼
              ┌──────────────────────────────┐
              │ GENERATE AND OUTPUT ONE      │──S30
              │ SCREEN OF IMAGE DATA         │
              └──────────────┬───────────────┘
                                │
                                ▼
                       ┌─────────────┐
                       │     END     │
                       └─────────────┘
```

## INTERNATIONAL SEARCH REPORT

| International application No. |
|---|
| PCT/JP2010/050801 |

A. CLASSIFICATION OF SUBJECT MATTER
*A61B10/00*(2006.01)i, *A61B8/08*(2006.01)i, *G01N21/17*(2006.01)i

According to International Patent Classification (IPC) or to both national classification and IPC

B. FIELDS SEARCHED

Minimum documentation searched (classification system followed by classification symbols)
A61B10/00, A61B8/08, G01N21/17

Documentation searched other than minimum documentation to the extent that such documents are included in the fields searched
Jitsuyo Shinan Koho          1922-1996   Jitsuyo Shinan Toroku Koho   1996-2010
Kokai Jitsuyo Shinan Koho    1971-2010   Toroku Jitsuyo Shinan Koho   1994-2010

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
|---|---|---|
| A | JP 2008-168038 A  (Olympus Medical Systems Corp.),<br>24 July 2008 (24.07.2008),<br>& US 2007/187632 A1     & EP 1810610 A1 | 1-5 |
| A | JP 2007-216001 A  (Olympus Medical Systems Corp.),<br>30 August 2007 (30.08.2007),<br>& US 2007/187632 A1     & EP 1810610 A1 | 1-5 |

☐ Further documents are listed in the continuation of Box C.    ☐ See patent family annex.

| | |
|---|---|
| * Special categories of cited documents:<br>"A" document defining the general state of the art which is not considered to be of particular relevance<br>"E" earlier application or patent but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |

| Date of the actual completion of the international search | Date of mailing of the international search report |
|---|---|
| 05 February, 2010 (05.02.10) | 16 February, 2010 (16.02.10) |

| Name and mailing address of the ISA/ | Authorized officer |
|---|---|
| Japanese Patent Office | |
| Facsimile No. | Telephone No. |

Form PCT/ISA/210 (second sheet) (April 2007)

## REFERENCES CITED IN THE DESCRIPTION

**Patent documents cited in the description**

• JP 2007216001 A

• JP 2009039709 A

**Non-patent literature cited in the description**

• **C. Kim ; K.H. Song ; L.V. Wang.** Sentinel lymph node detection ex vivo using ultrasound-modulated optical tomography. *J. Biomed. Opt.,* 2008, vol. 13 (2